# EUROPÄISCHE PATENTSCHRIFT

(11) **EP 4 277 776 B1**
(45) Veröffentlichungstag und Bekanntmachung des Hinweises auf die Patenterteilung: **17.12.2025**
(21) Anmeldenummer: 22702152.4
(22) Anmeldetag: 12.01.2022
(51) Int. Cl.: B29C 39/24, B29C 39/42, B29C 33/40, A61M 37/00, B29C 43/22, B29C 43/34, B29C 43/46

(54) **VERFAHREN UND SYSTEM ZUR HERSTELLUNG VON MIKROSTRUKTUREN**
METHOD AND SYSTEM FOR MANUFACTURING MICROSTRUCTURES
PROCEDE ET SYSTEME DE FABRICATION DE MICROSTRUCTURES

(30) Priorität: 12.01.2021 DE 102021100396
(43) Veröffentlichungstag der Anmeldung: 22.11.2023
(73) Patentinhaber: LTS Lohmann Therapie-Systeme AG, 56626 Andernach (DE)
(72) Erfinder: KULIK, Michael, 56218 Mülheim-Kärlich (DE); GRÜNWALD, Olga, 56077 Koblenz (DE); ROOS, Philip, 56220 Kettig (DE); TISSIN, Nikolaj, 53489 Sinzig (DE)
(74) Vertreter: dompatent
(86) Internationale Anmeldenummer: PCT/EP2022/050510
(87) Internationale Veröffentlichungsnummer: WO 2022/152734

(56) Entgegenhaltungen:
- EP-A1- 2 343 101
- EP-A1- 3 144 030
- WO-A1-2018/213605
- KR-A- 20170 011 578
- US-A1- 2017 050 010
- US-A1- 2019 030 309
- US-A1- 2019 351 204
- US-A1- 2020 197 679

## Beschreibung

Die Erfindung betrifft ein Verfahren zur Herstellung von Mikrostrukturen, insbesondere ein Verfahren zur Herstellung von Mikroarrays. Ferner betrifft die Erfindung ein System zur Herstellung von Mikrostrukturen, insbesondere ein System zur Herstellung von Mikroarrays.

Mikroarrays weisen eine Vielzahl von Mikronadeln auf, die üblicherweise in einem Trägerelement, wie in einem Patch, einem Pflaster oder dergleichen angeordnet bzw. mit diesem verbunden sind. Die Länge der Mikronadeln ist insbesondere so bemessen ist, dass sie beim Eindrücken in die Haut eines Patienten nur so weit in die Haut eindringen, dass Nerven und Gefäße möglichst nicht von den Nadelspitzen berührt werden. Die Nadeln weisen einen Wirkstoff, beispielsweise ein Medikament, auf. Der entsprechende Wirkstoff kann an einer Oberseite der Nadel aufgebracht sein oder in den Nadeln angeordnet sein. Bei in den Nadeln angeordnetem Wirkstoff sind die Nadeln oder Bestandteile der Nadeln aus einem sich in der Patientenhaut auflösenden Material hergestellt.

Die Herstellung von Mikroarrays erfolgt beispielsweise mit Hilfe von Silikonformen, die eine Vielzahl von Formöffnungen aufweisen. Zum Befüllen der Formöffnungen wird üblicherweise eine mit dem Wirkstoff versehene Flüssigkeit auf die Oberseite der Silikonmatrize aufgebracht. Nach dem Trocknen der Flüssigkeit wird ggf. eine weitere Flüssigkeit aufgebracht. Auf der Unterseite des in die Silikonmatrize eingebrachten Materials wird das Trägerelement aufgebracht, die Mikronadeln werden aus der Silikonmatrize entfernt und anschließend verpackt.

Die Herstellung von Mikroarrays ist aktuell sehr aufwendig und kostenintensiv.

Ferner kommt es beim Befüllen der, üblicherweise kegelförmigen oder pyramidenförmigen, Formöffnungen zu Problemen. Häufig werden die Formöffnungen nicht ausreichend mit Füllmaterial ausgefüllt. Beispielsweise kann es zu Hohlräumen und/der Lufteinschlüssen kommen. Dieses Problem tritt insbesondere in den Spitzen der, insbesondere kegelförmigen oder pyramidenförmigen, Formöffnungen auf. Durch derartig fehlerhafte Befüllung kann es bspw. zu Problemen bei der Applikation kommen, da z. B. die fehlerhaften Spitzen nicht ordnungsgemäß in die Haut eintreten und/oder es kann zu mangelhafter Wirkstoffdosierung aufgrund der in den Hohlräumen fehlenden Formulierung kommen.

Durch Verunreinigung und/oder Kontamination kommt es zu weiteren Problemen bei der heutigen Mikroarrayherstellung.

Stand der Technik zu der vorliegenden Anmeldung ist in EP 2 343 101 A1, US 2020/197679 A1, EP 3 144 030 A1, US 2017/050010 A1, US 2019/030309 A1, und KR 2017 0011578 A beschrieben.

Aufgabe der Erfindung ist es, ein Verfahren zur Herstellung von Mikrostrukturen, insbesondere von Mikroarrays zu schaffen, wobei die Skalierbarkeit verbessert ist und das vorzugsweise für die Herstellung hoher Stückzahlen geeignet ist. Ferner ist es Aufgabe der Erfindung, ein entsprechendes System zur Herstellung von Mikrostrukturen zu schaffen.

Die Lösung der Aufgabe erfolgt erfindungsgemäß durch ein Verfahren gemäß Anspruch 1 bzw. ein System gemäß Anspruch 14.

Bei dem erfindungsgemäßen Verfahren zur Herstellung von Mikrostrukturen handelt es sich insbesondere um ein Verfahren zur Herstellung von Mikroarrays. Ein, vorzugsweise erster, Schritt des Verfahrens besteht im Bereitstellen eines vorzugsweise flächigen Formelements. Das Formelement weist mindestens eine, vorzugsweise mehrere, Formöffnungen für die herzustellenden Mikrostrukturen auf. Bei der mindestens einen Formöffnung handelt es sich insbesondere um eine Negativform für die herzustellenden Mikrostrukturen. Die Formöffnung entspricht insbesondere einer Kavität. Die mindestens eine Formöffnung weist eine erste Öffnung und eine, insbesondere der ersten Öffnung gegenüberliegende, zweite Öffnung auf. Bevorzugt ist es somit, dass die Formöffnung beidseitig geöffnet ist. Es ist bevorzugt, dass die erste Öffnung und die zweite Öffnung die Formöffnung zur Umgebung hin öffnen. Vorzugsweis ist durch die erste Öffnung und die zweite Öffnung eine Fluidverbindung zwischen der Formöffnung und der Umgebung ausgebildet. Die Formöffnung verläuft vorzugsweise von einer Seite des flächigen Formelements zu einer der ersten Seite gegenüberliegenden Seite des Formelements. Ist das Formelement in bevorzugter Ausführung als Folie ausgebildet, verläuft die Formöffnung insbesondere von der einen Folienseite zu der anderen Folienseite. Es ist bevorzugt, dass es sich bei der mindestens einen Formöffnung um eine, vorzugsweise mittels Prägewalze hergestellte, geprägte Formöffnung handelt. Ein weiterer Schritt des Verfahrens besteht im Bereitstellen einer ersten Formulierung an der zweiten Öffnung. Es ist bevorzugt, dass die erste Formulierung mindestens einen Wirkstoff aufweist. Die erste Formulierung ist insbesondere zur Ausformung von Spitzen der Mikrostrukturen ausgeführt. Die Bereitstellung der ersten Formulierung erfolgt insbesondere derart, dass die Formulierung mit der zweiten Öffnung in Kontakt ist. Besonders bevorzugt ist es, dass die Bereitstellung derart erfolgt, dass die zweite Öffnung in die erste Formulierung eingetaucht ist. Ein weiterer Schritt des Verfahrens besteht im Erzeugen eines Unterdrucks und/oder Sogs in der Formöffnung. Der Unterdruck kann insbesondere auch als, vorzugweise nicht vollständiges, Vakuum bezeichnet werden. Unterdruck meint hierbei insbesondere ein negatives Druckverhältnis gegenüber dem Umgebungsdruck. Ein weiterer, insbesondere nächster Schritt, des Verfahrens besteht im Aufnehmen der ersten Formulierung durch die zweite Öffnung in die Formöffnung. Das Aufnehmen erfolgt hierbei zumindest teilweise aufgrund des Unterdrucks in der Formöffnung. Bevorzugt ist es hierbei, dass die erste Formulierung durch die zweite Öffnung eingesaugt wird. Zusätzlich zum Aufnehmen der ersten Formulierung aufgrund des Unterdrucks ist es bevorzugt, dass ein Aufnehmen aufgrund von Kapillarwirkung der ersten Formulierung in die Formöffnung erfolgt. Es ist bevorzugt, dass dieses Aufnehmen mittels Kapillarwirkung vor und/oder während des Aufnehmens mittels Unterdruck erfolgt. Das Aufnehmen der ersten Formulierung in die Formöffnung erfolgt insbesondere zur Ausbildung eines Teils der herzustellenden Mikrostrukturen. Bei dem Aufnehmen handelt es sich insbesondere um ein Einziehen oder Einsaugen.

In bevorzugter Ausführung erfolgt das Erzeugen des Unterdrucks in der Formöffnung durch ein Erweitern eines Volumens der Formöffnung. Bei dem zu erweiternden Volumen handelt es sich insbesondere um das Volumen zwischen der ersten und der zweiten Öffnung der Formöffnung. Es ist bevorzugt, dass das Erweitern des Volumens der Formöffnung derart erfolgt, dass die daraus resultierende Volumenerweiterung im Wesentlichen dem aufzunehmenden Volumen der ersten Formulierung entspricht. Insbesondere erfolgt ein Erweitern des Volumens der Formöffnung um mindestens 3 %, bevorzugt um mindestens 5 %, besonders bevorzugt um mindestens 10 %, wobei weiter bevorzugt ein Erweitern des Volumens der Formöffnung um mindestens 20 % erfolgt.

Ein weiterer Schritt des Verfahrens besteht in einem Verringern des Volumens der Formöffnung, wobei dieser Schritt vor dem Erzeugen des Unterdrucks in der Formöffnung, insbesondere vor dem Erweitern des Volumens der Formöffnung, durchgeführt wird. Der Schritt der Bereitstellung der ersten Formulierung erfolgt vorzugsweise vor und/oder nach dem Verringern des Volumens. Es ist bevorzugt, dass das Verringern des Volumens der Formöffnung derart erfolgt, dass die daraus resultierende Volumenverringerung im Wesentlichen dem aufzunehmenden Volumen der ersten Formulierung entspricht. Insbesondere erfolgt ein Verringern des Volumens der Formöffnung um mindestens 3 %, bevorzugt um mindestens 5 %, besonders bevorzugt um mindestens 10 %, wobei weiter bevorzugt ein Verringern des Volumens der Formöffnung um mindestens 20 % erfolgt.

Das Verringern des Volumens der Formöffnung erfolgt durch ein, zumindest teilweises, Komprimieren des Formelements. Beim Komprimieren handelt es sich insbesondere um ein Pressen und/oder ein Längsdehnen. Zum Komprimieren ist es bevorzugt, dass das Formelement an der Seite der ersten Öffnung, bevorzugt an beiden Seiten der beiden Öffnungen, gepresst wird und/oder das Formelement, insbesondere in Längsrichtung, gestreckt wird.

Es ist bevorzugt, dass das Komprimieren des Formelements mittels mindestens einer Walze erfolgt. Bei der Walze handelt es sich insbesondere um eine Presswalze. Die mindestens eine Presswalze presst hierbei auf zumindest eine Fläche, insbesondere auf die Fläche der ersten Öffnung des Formelements. Besonders bevorzugt erfolgt das Komprimieren mittels zwei Walzen beidseitig des Formelements. Es ist bevorzugt, dass die beiden Walzen in Walzrichtung versetzt angeordnet sind.

Bevorzugt besteht ein weiterer Schritt des Verfahrens in einem Anordnen eines Hilfselements an der die erste Öffnung aufweisenden Seite des Formelements. Das Hilfselement wird hierbei mit dieser Seite in Kontakt gebracht, insbesondere mit dieser, vorzugsweise adhäsiv, verbunden. Das Hilfselement weist vorzugsweise eine Hilfsfolie auf, besteht insbesondere daraus. Das Anordnen des Hilfselements erfolgt bevorzugt vor dem Erzeugen des Unterdrucks in der Formöffnung, besonders bevorzugt vor dem Erweitern des Volumens der Formöffnung. Insbesondere erfolgt das Anordnen des Hilfselements vor oder während des Verringerns des Volumens der Formöffnung.

Bevorzugt besteht ein weiterer Schritt des Verfahrens in einem Verschließen der ersten Öffnung der Formöffnung. Insbesondere erfolgt ein im Wesentlichen luftdichtes Verschließen der ersten Öffnung. Es ist bevorzugt, dass das Verschließen mit dem Hilfselement erfolgt, so dass das Hilfselement die erste Öffnung der Formöffnung abdichtet. Der Schritt des Verschließens der ersten Öffnung erfolgt vor Erzeugen des Unterdrucks in der Formöffnung. Insbesondere erfolgt das Verschließen der ersten Öffnung nach Verringern des Volumens der Formöffnung, vorzugsweise nach dem Komprimieren des Formelements.

Ein bevorzugter weiterer Schritt des Verfahrens besteht in einem Entfernen des Hilfselements, vorzugsweise vom Formelement. Dieses Entfernen erfolgt nach dem Aufnehmen der ersten Formulierung in der Formöffnung. Es ist bevorzugt, dass das Entfernen durch ein Abziehen des Hilfselements erfolgt.

Es ist bevorzugt, dass die mindestens eine Formöffnung zylinderförmig oder kegelförmig ist. Die Zylinder- oder Kegelform weist hierbei insbesondere einen runden, dreieckigen oder viereckigen, besonders bevorzugt quadratischen Querschnitt auf. Es ist bevorzugt, dass sich die Kegelform von der ersten Öffnung zur zweiten Öffnung der Formöffnung verjüngt. Möglich ist es, dass die Kegelform als Kegelstumpf ausgebildet ist. Eine Kegelform mit eckigem Querschnitt kann auch als Pyramidenform beschrieben werden.

Die zweite Öffnung weist vorzugsweise eine geringere Querschnittsfläche als die erste Öffnung auf. Bevorzugt verjüngt sich der Querschnitt der Formöffnung von der ersten Öffnung in Richtung der zweiten Öffnung.

Bevorzugt erfolgt das Verringern des Volumens der Formöffnung, insbesondere das zumindest teilweise Komprimieren des Formelements, durch ein Biegen des Formelements. Das Biegen erfolgt insbesondere entlang einer Querrichtung des Formelements, sodass insbesondere eine Biegekante senkrecht zur Längsrichtung des Formelements liegt. Besonders bevorzugt erfolgt das Biegen in Richtung der ersten Öffnung und/oder in einer Richtung entgegengesetzt zur Richtung der Verjüngung der Formöffnung, insbesondere bei kegelförmiger Formöffnung. Somit liegt bevorzugt eine gestauchte Faser des Formelements auf der Seite der ersten Öffnung und/oder auf der Seite, auf der die Formöffnung vergrößert. Das Biegen erfolgt insbesondere in einem Winkel von 0° bis 180°, bevorzugt von 10° bis 90°, besonderes bevorzugt von 30° bis 60°. Durch das Biegen weist das Formelement insbesondere eine neutrale Faser sowie eine gestauchte Faser auf der Seite des Formelements, in die das Biegen erfolgt, sowie eine gestreckte Faser auf der der gestauchten Faser gegenüberliegenden Seite auf. Bevorzugt liegt somit auf der Seite der gestauchten Faser des Formelements ein gestauchter Bereich und auf der Seite der gestreckten Faser des Formelements ein gestreckter Bereich vor. Durch das Biegen kommt es bevorzugt dazu, dass sich der gestauchte Bereich, insbesondere die gestauchte Faser, des Formelements verkleinert. Somit verkleinert sich insbesondere auch die Formöffnung, vorzugsweise das Volumen der Formöffnung, in diesem Bereich des Formelements. Ebenso kommt es somit durch das Biegen bevorzugt dazu, dass sich der gestreckte Bereich, insbesondere die gestreckte Faser des Formelements vergrößert. Somit vergrößert sich insbesondere auch die Formöffnung, vorzugsweise das Volumen der Formöffnung, in diesem Bereich des Formelements. In bevorzugter Ausführung, insbesondere in Ausführung der Formöffnung mit Kegelform, weist die Formöffnung auf Seite der gestauchten Faser ein, insbesondere wesentlich, größeres Volumen als auf der Seite mit gestreckter Faser auf. Durch das Biegen kommt es somit bevorzugt dazu, dass sich das Volumen auf Seite der gestauchten Faser stärker verkleinert, als sich das Volumen auf Seite der gestreckten Faser vergrößert. Insgesamt liegt somit vorzugsweise eine Verkleinerung des gesamten Volumens der Formöffnung durch das Biegen vor.

Das Biegen erfolgt bevorzugt mit der mindestens einen Walze. Es handelt sich bei der mindestens einen Walze sodann bevorzugt um eine Biegewalze.

Das Erweitern des Volumens erfolgt insbesondere durch ein Zurückversetzen des Formelements, insbesondere in die Ursprungsform. Besonders bevorzugt handelt es sich bei dem Zurückversetzen um ein Rückbiegen des durch das Biegen gebogenen Formelements und/oder um ein Entspannen des komprimierten, insbesondere gepressten und/oder längsgedehnten Formelements.

Es ist bevorzugt, dass das Formelement eine Folie aufweist, insbesondere daraus besteht.

Besonders bevorzugt ist es, dass das Formelement TPU, PC oder PETG aufweist, insbesondere daraus besteht.

Das Formelement ist vorzugsweise komprimierbar. Es ist bevorzugt, dass das Formelement elastisch ausgeführt ist.

Nach dem Schritt des Aufnehmens der ersten Formulierung erfolgt vorzugsweise ein Bereitstellen einer zweiten Formulierung an der ersten Öffnung. Es ist bevorzugt, dass die zweite Formulierung wirkstofffrei ist. Möglich ist es jedoch auch, dass die zweite Formulierung mindestens einen Wirkstoff aufweist. Nach dem Bereitstellen der zweiten Formulierung erfolgt ein Aufnehmen der zweiten Formulierung durch die erste Öffnung in die Formöffnung. Bevorzugt ist es, dass hierbei die Formöffnung, insbesondere das restliche Volumen der Formöffnung, vollständig mit der zweiten Formulierung ausgefüllt wird. Insbesondere verbindet sich die zweite Formulierung mit der ersten Formulierung, vorzugsweise stoffschlüssig. Bevorzugt erfolgt das Aufnehmen der zweiten Formulierung durch ein Eindrücken, insbesondere Einpressen. Vorzugsweise erfolgt das Eindrücken mittels Walze. Es ist bevorzugt, dass beim Schritt des Aufnehmens die Formulierung auf der einen Seite mit der ersten Öffnung in Kontakt ist und auf der anderen Seite durch ein Formulierungselement, insbesondere das unten beschriebene zweite Formulierungselement, abgedeckt wird. Bevorzugt liegt der Druck zum Eindrücken der zweiten Formulierung, insbesondere die Walze, an diesem Formulierungselement an.

Bevorzugt erfolgt das Bereitstellen der ersten Formulierung mittels eines ersten Formulierungselements. Das erste Formulierungselement weist vorzugsweise eine Folie auf, besteht insbesondere daraus. Das erste Formulierungselement weist die erste Formulierung auf. Bevorzugt ist die erste Formulierung, insbesondere tröpfchenweise, auf dem ersten Formulierungselement angeordnet. Es ist bevorzugt, dass das erste Formulierungselement eine, vorzugsweise eine Vertiefung aufweisende, Formulierungsaufnahme aufweist. Mit anderen Worten handelt es sich bei dieser Formulierungsaufnahme um eine Art schalenförmige Form im Formulierungselement, in der sich die Formulierung befindet. Alternativ oder zusätzlich zum Bereitstellen der ersten Formulierung mittels des ersten Formulierungselements erfolgt das Bereitstellen der zweiten Formulierung mittels eines zweiten Formulierungselements, das die zweite Formulierung aufweist. Das zweite Formulierungselement ist insbesondere entsprechend mit einem oder mehreren Merkmalen des ersten Formulierungselements ausgeführt.

Nach dem Aufnehmen der ersten Formulierung und/oder nach dem Aufnehmen der zweiten Formulierung erfolgt insbesondere ein Trocknen. Das Trocknen der ersten und/oder zweiten Formulierung erfolgt insbesondere durch die erste Öffnung der Formöffnung. Das Trocknen erfolgt insbesondere mittels, bevorzugt warmem, Luftstrom, der insbesondere direkten Kontakt mit der ersten und/oder der zweiten Formulierung hat. Es ist bevorzugt, dass durch das Trocknen ein Erstarren der ersten und/oder zweiten Formulierung zu Mikrostrukturen erfolgt. Zusätzlich oder alternativ zum Luftstrom kann das Trocknen mittels Wärmestrahlung, insbesondere Infrarotstrahlung, erfolgen.

Ein bevorzugter weiterer Schritt des Verfahrens besteht in einem, insbesondere messtechnischen, Prüfen der ersten und/oder zweiten Formulierung. Das Prüfen erfolgt insbesondere mittels optischer Prüfvorrichtung. Es ist bevorzugt, dass die optische Prüfvorrichtung eine Kamera aufweist. Das Prüfen erfolgt nach dem Aufnehmen der ersten und/oder nach dem Aufnehmen der zweiten Formulierung. Besonders bevorzugt ist es, dass das Prüfen nach oder vor dem Schritt des Trocknens erfolgt.

Bevorzugt erfolgt ein Entformen der zumindest teilweise zur Mikrostruktur erstarrten ersten Formulierung sowie vorzugsweise der mit der ersten Formulierung verbundenen zweiten Formulierung. Das Entformen erfolgt an und durch die erste Öffnung der Formöffnung. Bevorzugt erfolgt das Entformen nach dem Trocknen, besonders bevorzugt nach dem Prüfen. Das Entformen erfolgt vorzugsweise an der zweiten Formulierung.

Das Entformen erfolgt vorzugsweise mittels eines Deckelements. Das Deckelement weist insbesondere eine Deckfolie auf, besteht insbesondere daraus. Das Deckelement wird mit der ersten Formulierung oder der zweiten Formulierung verbunden. Es ist bevorzugt, dass die Verbindung stoffschlüssig, insbesondere adhäsiv, erfolgt. Alternativ oder zusätzlich zum Entformen mittels Deckelement erfolgt das Entformen durch ein Entfernen, insbesondere ein Abziehen des Formelements. Bevorzugt ist es, dass es sich bei dem Deckelement um das zweite Formulierungselement handelt, sodass das Entformen mittel zweitem Formulierungselement erfolgt.

Bevorzugt erfolgt ein Verpacken der entformten Mikrostruktur. Es ist bevorzugt, dass das Verpacken mittels eines Blisterelements erfolgt. Besonders bevorzugt erfolgt das Verpacken mittels Blisterfolie.

Das Verfahren wird insbesondere mit einem System mit einem oder mehreren Merkmalen des nachstehend beschriebenen Systems durchgeführt.

Bei dem erfindungsgemäßen System zur Herstellung von Mikrostrukturen handelt es sich insbesondere um ein System zur Herstellung von Mikroarrays. Bei dem System handelt es sich insbesondere um eine Vorrichtung. Das System ist vorzugsweise zur Durchführung des oben beschriebenen Verfahrens ausgeführt. Es ist besonders bevorzugt, dass das System eines oder mehrere der oben beschriebenen Merkmale, insbesondere der dort beschriebenen Vorrichtungsmerkmale, aufweist. Das System weist ein Formelement auf. Das Formelement ist insbesondere komprimierbar oder elastisch. Das Formelement weist mindestens eine Formöffnung für die herzustellenden Mikrostrukturen auf. Bei der mindestens einen Formöffnung handelt es sich insbesondere um eine Negativform für die herzustellenden Mikrostrukturen. Die Formöffnung entspricht insbesondere einer Kavität. Vorzugsweise weist die Formöffnung eine erste Öffnung und eine, insbesondere der ersten Öffnung gegenüberliegende, zweite Öffnung auf. Die Formöffnung ist insbesondere beidseitig geöffnet, sodass das Formelement mindestens eine durchgehende Formöffnung aufweist. Es ist bevorzugt, dass die erste Öffnung und die zweite Öffnung die Formöffnung zur Umgebung hin öffnen. Vorzugsweis ist durch die erste Öffnung und die zweite Öffnung eine Fluidverbindung zwischen der Formöffnung und der Umgebung ausgebildet. Ferner weist das System eine Komprimiervorrichtung auf. Bei der Komprimiervorrichtung handelt es sich insbesondere um eine Press- und/oder Biegevorrichtung. Die Komprimiervorrichtung weist eine Walze, besonders bevorzugt eine Presswalze und/oder Biegewalze, auf, besteht insbesondere daraus. Die Komprimiervorrichtung ist zum Komprimieren des Formelements ausgeführt. Insbesondere ist die Komprimiervorrichtung zum pressenden Einwirken auf das Formelement und/oder zum Biegen des Formelements angeordnet und/oder ausgeführt. In bevorzugter Ausführung ist die Komprimiervorrichtung zur Verringerung des Volumens der Formöffnung durch das Komprimieren ausgeführt.

Es ist bevorzugt, dass die Komprimiervorrichtung zwei sich gegenüberliegende Walzen, insbesondere Presswalzen und/oder Biegewalzen, aufweist. Die Walzen sind insbesondere derart angeordnet, dass das Formelement zwischen den Walzen komprimierbar, insbesondere pressbar und/oder biegbar ist. Das Formelement befindet sich vorzugsweise zwischen den Walzen. Insbesondere sind die Walzen sind in zueinander und/oder in Walzenführrichtung versetzt angeordnet.

Es ist bevorzugt, dass das System einen, vorzugsweise aseptischen, Isolator aufweist, in dem zumindest die Komprimiervorrichtung und zumindest ein Teil des Formelements angeordnet ist.

Bei dem Formelement des erfindungsgemäßen Verfahrens und/oder des erfindungsgemäßen Systems handelt es sich insbesondere um ein Formelement gemäß DE 10 2020 125 484 A1.

Nachfolgend wird die Erfindung anhand bevorzugter Ausführungsformen unter Bezugnahme auf die anliegenden Zeichnungen näher erläutert.

Es zeigen:
- Fig. 1a-1d: schematische, geschnittene Seitenansichten von Herstellungszuständen zur Darstellung des erfindungsgemäßen Verfahrens, wobei Fig. 1b ebenfalls eine erfindungsgemäße Ausführung eines Systems zur Herstellung von Mikrostrukturen zeigt,
- Fig. 2a-2c: schematische, geschnittene Seitenansichten von Herstellungszuständen zur Darstellung des erfindungsgemäßen Verfahrens, wobei Fig. 2c ebenfalls eine erfindungsgemäße Ausführung eines Systems zur Herstellung von Mikrostrukturen zeigt,
- Fig. 3: eine schematische, geschnittene Seitenansicht eines Herstellungszustands zur Darstellung des erfindungsgemäßen Verfahrens, wobei ebenfalls eine erfindungsgemäße Ausführung eines Systems zur Herstellung von Mikrostrukturen gezeigt ist,
- Fig. 4: eine schematische, geschnittene Seitenansicht eines Herstellungszustands zur Darstellung einer Ausführung des erfindungsgemäßen Verfahrens, und
- Fig. 5: eine schematische Seitenansicht zur Darstellung einer Ausführung des erfindungsgemäßen Verfahrens mit einer Darstellung einer weiteren erfindungsgemäßen Ausführungsform eines Systems zur Herstellung von Mikrostrukturen.

Ähnliche oder identische Bauteile oder Elemente werden in den Figuren mit gleichen Bezugszeichen bzw. Variationen davon (12, 12' und 12") identifiziert. Insbesondere zur verbesserten Übersichtlichkeit werden, vorzugsweise bereits identifizierte, Elemente nicht in allen Figuren mit Bezugszeichen versehen.

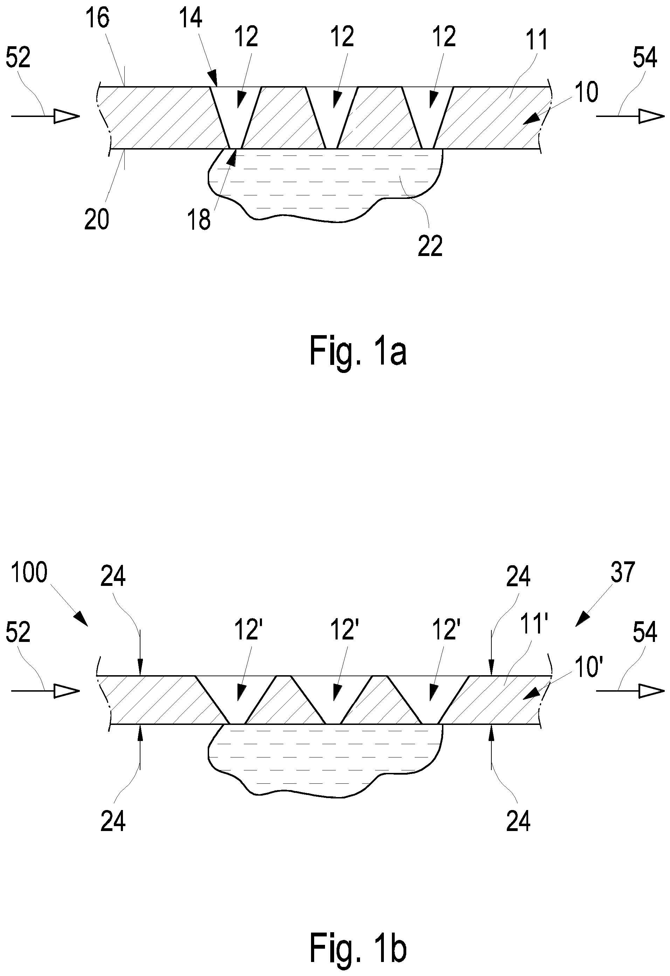

Fig. 1a zeigt ein Formelement 10, das hier insbesondere als elastische Folie 11 ausgeführt ist. An der dargestellten Oberseite weist die Folie 11 eine erste Seite 16 sowie gegenüberliegend eine zweite Seite 20 auf. Durch die Folie 11 verlaufen kegel- oder pyramidenförmige Formöffnungen 12. An der ersten Seite 16 weisen die Formöffnungen 12 eine erste Öffnung 14 auf. Die Formöffnungen 12 verjüngen sich, ausgehend von der ersten Seite 16 zur zweiten Seite 20, wobei an der zweiten Seite 20 eine zweite Öffnung 18 ausgebildet ist. An der zweiten Seite 20 ist eine erste, vorzugsweise wirkstoffhaltige, Formulierung 22 in Kontakt mit den zweiten Öffnungen 18 der Formöffnungen 12 angeordnet. Möglich ist es (nicht dargestellt), dass ein Teil der Formulierung 22 aufgrund von Kapillareffekten durch die zweiten Öffnungen 18 in die Formöffnungen 12 aufgenommen wird.

Mit Pfeil 52 ist eine Zuführung, mit Pfeil 54 eine Abführung des Formelements dargestellt, so dass das Verfahren vorzugsweise als Fließprozess und/oder als Rollenprozess ausgeführt werden kann.

Fig. 1b zeigt einen zweiten Zustand der Ausführung aus Fig. 1a. Schematisch dargestellt durch die Pfeile 24 ist ein Druck auf das Formelement 10 bzw. die Folie 11 ausgeübt worden. Bevorzugt ist das Formelement zusammengepresst worden. Aufgrund der Druckausübung erfolgt eine Komprimierung des Formelements 10, so dass das Volumen der Formöffnungen 12' im Vergleich zur Ausführung aus Fig. 1a verringert wurde. Die Druckausübung kann mittels Komprimiervorrichtung 37, beispielsweise Pressvorrichtung oder Walzvorrichtung, einseitig oder beidseitig des Formelements 10 erfolgen.

Dargestellt ist in Fig. 1b auch ein System 100 zur Herstellung von Mikrostrukturen mit Formelement 10 und Komprimiervorrichtung 37. Bei dieser Komprimiervorrichtung 37 handelt es sich insbesondere um eine Pressvorrichtung.

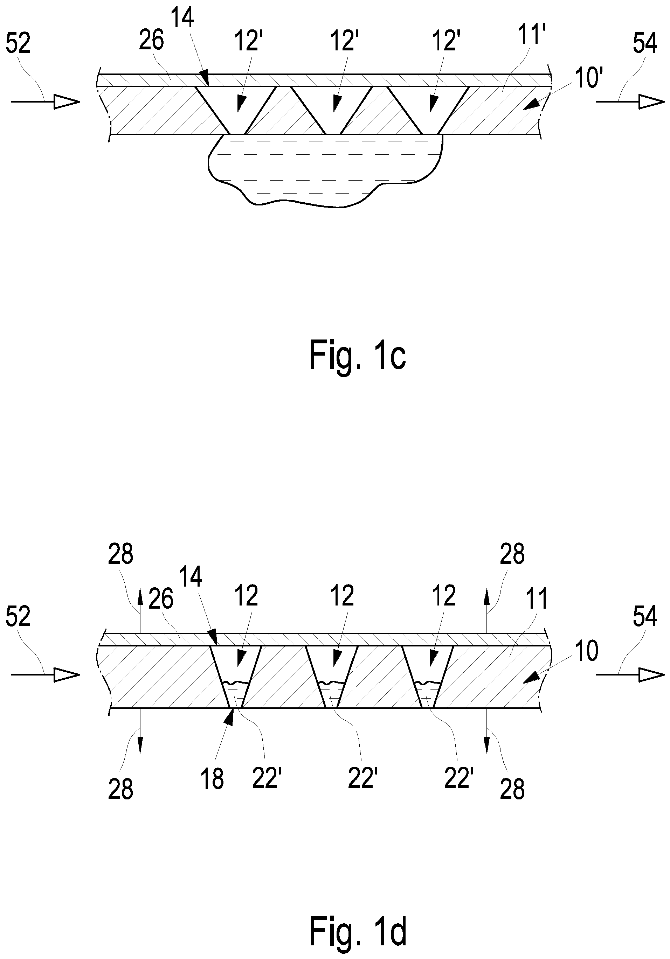

Fig. 1c zeigt einen weiteren Zustand in Anlehnung an die Ausführungen aus den Figuren 1a und 1b.

An der ersten Seite 16 des Formelements 10 wurde ein Hilfselement 26, insbesondere ausgebildet als Folie, angeordnet. Das Hilfselement 26 verschließt hierbei die ersten Öffnungen 14 der volumenreduzierten Formöffnungen 12'.

Fig. 1d zeigt einen weiteren Zustand der Ausführungen aus den Figuren 1a bis 1c. Im Gegensatz zu den Ausführungen aus den Figuren 1b bis 1c hat sich das Formelement 10 wieder ausgedehnt, insbesondere entspannt (dargestellt über Pfeile 28). Besonders bevorzugt ist es, dass sich das Formelement 10 elastisch zurückgedehnt hat. Somit hat sich auch das Volumen der Formöffnungen 12 wieder erweitert. Durch die Erweiterung des Volumens der Formöffnungen 12 entsteht ein Unterdruck in den Formöffnungen 12. Da die erste Öffnung 14 der Formöffnungen 12 verschlossen ist, entsteht ein Sog an den zweiten Öffnungen 18. Durch den Sog erfolgte ein Aufnehmen, insbesondere ein Einsaugen, der ersten Formulierung 22 in die Formöffnungen 12, so dass die Formöffnungen 12 nun teilweise mit Formulierungen 22' gefüllt sind. Möglich ist es auch, dass durch entsprechende Dimensionierung die gesamten Formöffnungen 12 mit der ersten Formulierung 22 gefüllt werden. Möglich ist, es dass durch ein Aushärten der Formulierungen 22' Mikrostrukturen, insbesondere Mikronadeln, im Formelement 10 entstehen.

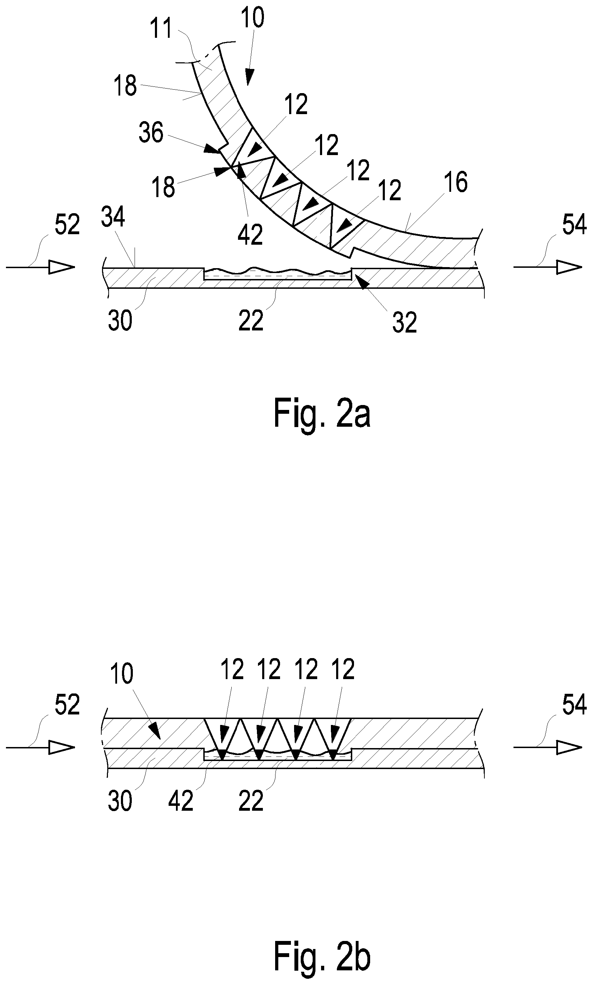

Das Formelement 10 aus Fig. 2a entspricht im Wesentlichen dem Formelement 10 der Figuren 1a bis 1d. Im Unterschied zu der Ausführung der Figuren 1a bis 1d befinden sich die Spitzen der Formöffnungen 12 in Fig. 2a in einem Überstand 36 des Formelements 10. Die zweiten Öffnungen 18 der Formelemente 12 aus Fig. 2a münden somit in diesen Überstand 36. Die Bereitstellung der ersten Formulierung 22 erfolgt in einer Vertiefung 32 eines als Folie 30 ausgebildeten Formulierungselements 30. Die Vertiefung 32 befindet sich hierbei an einer ersten Seite 34 des Formulierungselements 30, wobei es bevorzugt ist, dass die Vertiefung 32 im Wesentlichen einer Negativform des Überstands 36 entspricht.

In Fig. 2a ist dargestellt, dass die Folie 11 des Formelements 10 und die Folie 30 des Formulierungselements 30 zusammengeführt, insbesondere in Verbindung gebracht werden (Zusammenführung von links nach rechts dargestellt).

Fig. 2b zeigt einen weiteren Zustand gemäß der Ausführung aus Fig. 2a. Formelement 10 und Formulierungselement 30 sind hierbei in Verbindung miteinander gebracht, so dass Überstand 36 in Vertiefung 32 angeordnet ist. Aufgrund von Kapillareffekten ist ein Teil der Formulierung 22 in die Spitzen 42 der Formöffnungen 12 durch zweite Öffnungen 18 eingetreten.

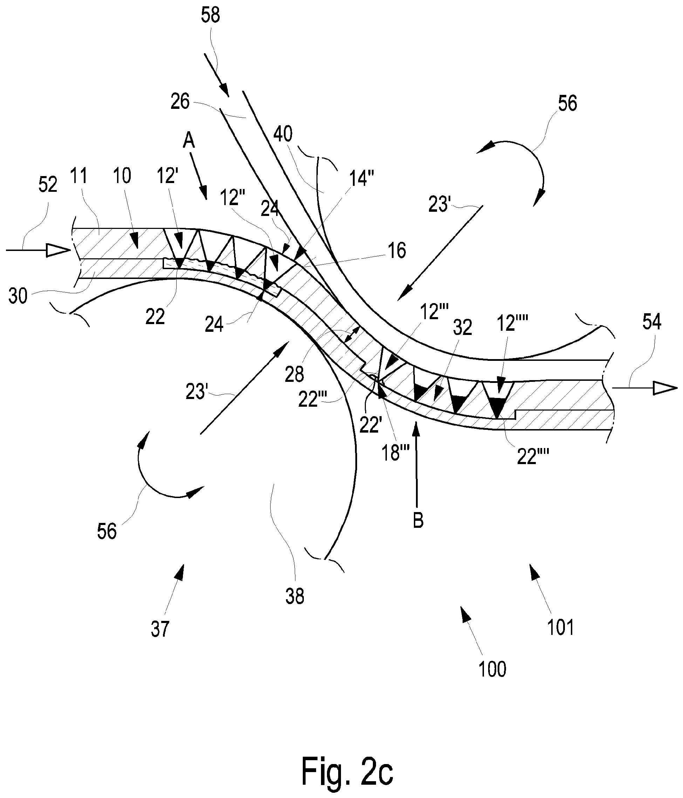

Fig. 2c zeigt einen weiteren Zustand, wobei es bevorzugt ist, dass die Ausführung aus Fig. 2b von links in Pfeilrichtung 52 zugeführt wird.

Durch Komprimiervorrichtung 37, die hier zwei versetzte Walzen 38, 40 aufweist, erfolgt eine Druckausübung bzw. ein Zusammenpressen des Formelements 10 (dargestellt durch Pfeile 23'). Das Formelement mit damit verbundenem Formulierungselement 30 verläuft dargestellt von links nach rechts (in Pfeilrichtung der Pfeile 52, 54). Die Drehrichtung der Walzen 38, 40 ist über Pfeile 56 dargestellt. Dargestellt ist es bevorzugt, dass Walze 38 im Uhrzeigersinn und Walze 40 gegen den Uhrzeigersinn dreht. Aufgrund der Druckausübung der Walzen 38, 40 auf das Formelement 10 (entsprechend Pfeilen 23') sowie einer Längsdehnung, insbesondere durch die Zugkraft der Walzen 38,40, erfolgt eine Komprimierung des elastischen Formelements 10. Aufgrund dieser Komprimierung verringert sich das Volumen der Formöffnungen 12 des Formelements 10. Diese Volumenänderung der Formöffnungen 12 ist im Bereich A dargestellt. Hier wurde im Bereich der Formöffnung 12" das Formelement bereits komprimiert, so dass das Volumen der Formöffnung 12" verringert ist. Im Bereich der Formöffnung 12' hingegen wurde das Formelement 10 noch nicht komprimiert, so dass das Volumen der Formöffnungen 12' im Ausgangszustand ist.

Dargestellt wird im Bereich zwischen den Walzen 38, 40 ein als Folie ausgebildetes Hilfselement 26 in Richtung des Pfeils 58 zugeführt. Das Hilfselement 26 bedeckt hierbei die erste Seite 16 des komprimierten Formelements 10, so dass die ersten Öffnungen 14" der volumenverringerten Formöffnungen 12" verschlossen werden.

Nach dem Durchlaufen des Bereichs zwischen den Walzen 38, 40 entspannt sich das Formelement, dehnt sich bevorzugt elastisch zurück. Dies ist im Bereich B dargestellt. Aufgrund der elastischen Verformung des Formelements 10 erweitert sich das Volumen der Formöffnungen 12. Hierdurch kommt es zu einem Unterdruck in den Formöffnungen 12. Durch den Unterdruck erfolgt ein Einsaugen der ersten Formulierung 22 durch die zweiten Öffnungen 18 der Formöffnungen 12. Die rechts dargestellte Formöffnung 12ʺʺ hat sich hierbei bereits vollständig erweitert, so dass ein Teil der Formöffnungen 12ʺʺ bereits vollständig mit der ersten Formulierung 22ʺʺ befüllt ist. Das Volumen der Formöffnungen 12‴ hingegen ist noch nicht vollständig erweitert, so dass erst ein Teil der Formulierung 22‴ aufgenommen wurde, während ein Teil der Formulierungen 22' in der Vertiefung 32 verbleibt. Bei einem Weiterführen und vollständigen Erweitern der Formöffnungen 12‴ kann sodann auch diese restliche Formulierung 22' aufgenommen werden.

Dargestellt ist in Fig. 2c ebenfalls ein Subsystem 101 zur Herstellung von Mikrostrukturen mit Komprimiervorrichtung 37, Formelement 10, Hilfselement 26 und Formulierungselement 30. Bei der Komprimiervorrichtung 37 handelt es sich insbesondere um eine Pressvorrichtung. Diese Subsystem 101 entspricht hierbei einer erfindungsgemäßen Ausführungsform eines Systems 100 zur Herstellung von Mikrostrukturen.

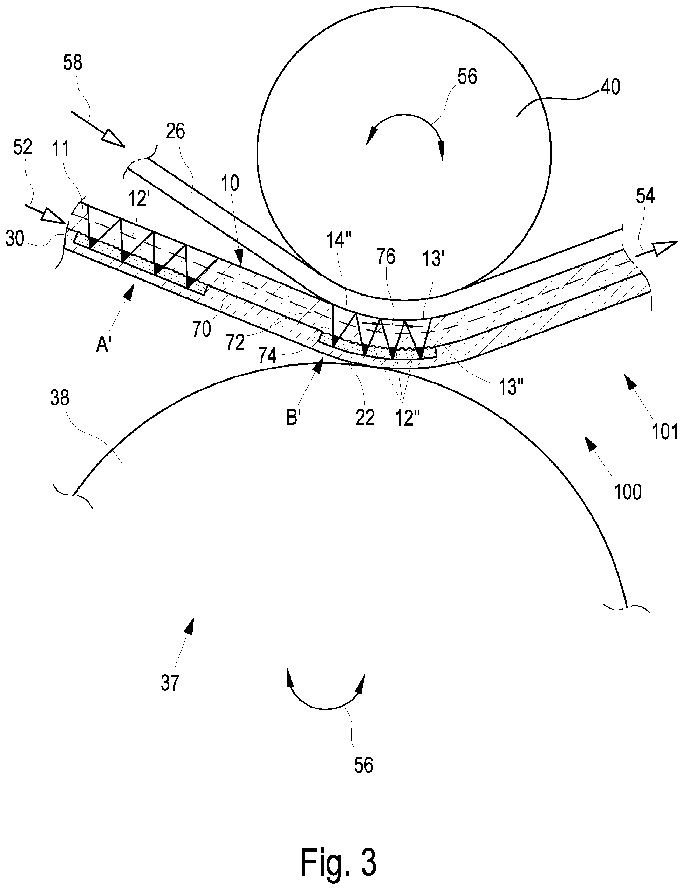

Fig. 3 zeigt ebenfalls einen Herstellungszustand, wobei es bevorzugt ist, dass die Ausführung aus Fig. 2b von links in Pfeilrichtung 52 zugeführt wird. Bei der Ausführung der Fig. 3 handelte es sich insbesondere um eine Alternative zu der Ausführung aus Fig. 2c. Die Darstellung der Fig. 3 ist an die Darstellung aus Fig. 2c angelehnt.

Durch Komprimiervorrichtung 37, die hier zwei versetzte Walzen 38, 40 aufweist, erfolgt ein Biegen des Formelements 10 um die Walze 40. Das Formelement mit damit verbundenem Formulierungselement 30 verläuft dargestellt von links nach rechts (in Pfeilrichtung der Pfeile 52, 54). Die Drehrichtung der Walzen 38, 40 ist über Pfeile 56 dargestellt. Dargestellt ist es bevorzugt, dass Walze 38 im Uhrzeigersinn und Walze 40 gegen den Uhrzeigersinn dreht. Durch das Biegen des Formelements 10 liegt (dargestellt im Bereich B') ein gestauchter Bereich 72, auch als gestauchte Faser zu bezeichnen, und ein gestreckter Bereich 74, auch als gestreckte Faser zu bezeichnen, des Formelements 10, beidseitig der neutralen Faser 70, vor. Durch die Stauchung (dargestellt durch Pfeil 76) kommt es zu einer Verringerung des Volumens 13' im gestauchten Bereich der Formöffnung 12". Im gestreckten Bereich der Formöffnung 12" kommt es hingegen zu einer Vergrößerung des Volumens 13". Da der Volumenanteil des gestauchten Bereichs, im Vergleich zu dem Teil, der im gestreckten Bereich liegt, wesentlich größer ist, erfolgt in Gänze eine Volumenverkleinerung der Formöffnung 12".

Im Bereich A' wurde hingegen das Formelement 10 (noch) nicht gebogen, so dass das Volumen der Formöffnungen 12' (noch) im Ausgangszustand ist.

Dargestellt wird im Bereich zwischen den Walzen 38, 40 ein als Folie ausgebildetes Hilfselement 26 in Richtung des Pfeils 58 zugeführt. Das Hilfselement 26 bedeckt hierbei die erste Seite 16 des komprimierten Formelements 10, so dass die ersten Öffnungen 14" der volumenverringerten Formöffnungen 12" verschlossen werden.

Nach dem Durchlaufen des Bereichs zwischen den Walzen 38, 40 wird das Formelement 10 in den, nicht gebogenen, Ausgangszustand zurückgeführt. Hierdurch erfolgt nun, entsprechend gegenteilig zur Gesamtvolumenverkleinerung im Bereich B', eine Gesamtvolumenvergrößerung der Formöffnung. Hierdurch kommt es zu einem Unterdruck in den Formöffnungen 12. Durch den Unterdruck erfolgt ein Einsaugen der ersten Formulierung 22 durch die zweiten Öffnungen 18 der Formöffnungen 12.

Dargestellt ist in Fig. 3 ebenfalls ein Subsystem 101 zur Herstellung von Mikrostrukturen mit Komprimiervorrichtung 37, Formelement 10, Hilfselement 26 und Formulierungselement 30. Bei dieser Komprimiervorrichtung 37 handelt es sich insbesondere um eine Biegevorrichtung. Diese Subsystem 101 entspricht hierbei einer erfindungsgemäßen Ausführungsform eines Systems 100 zur Herstellung von Mikrostrukturen.

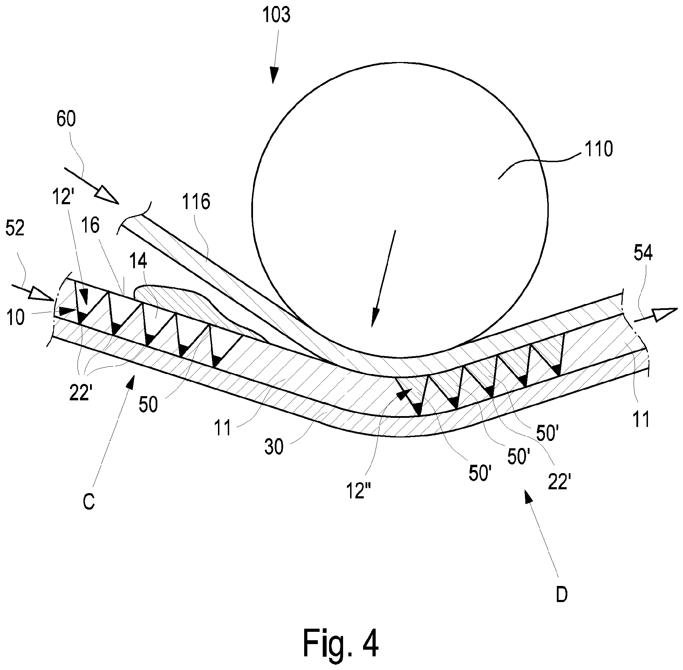

Fig. 4 zeigt einen weiteren Zustand gemäß einem erfindungsgemäßen Verfahren zur Herstellung von Mikrostrukturen. Von links in Richtung des Pfeils 52 wird ein Formelement 10 zugeführt. Das Formelement 10 weist mehrere Formöffnungen 12 auf. Die Unterseite des Formelements 10 ist mit einem Formulierungselement 30 verbunden. Bevorzugt ist es, dass das Formelement 10 gemäß den Ausführungen der Figuren 1d oder 2c ausgeführt ist, wobei jedoch das Hilfselement 26 entfernt, insbesondere abgezogen wurde.

Im dargestellten Bereich C sind die Formöffnungen 12' bereits teilweise mit einer ersten Formulierung 22' befüllt, wobei jedoch der Bereich der Pyramidenbasis der Formöffnungen 12' leer ist. Auf die erste Seite 16 des Formelements 10 im Bereich der ersten Öffnungen 14 wurde eine, vorzugweise wirkstofffreie, Formulierung 50 auf das Formelement aufgetragen.

In Richtung des Pfeils 60 wird ein zweites Formulierungselement 116, das insbesondere als Folie ausgebildet ist, zugeführt, das die zweite Formulierung bedeckt. Über die Walze 110 wird ein Druck auf das zweite Formulierungselement 116 ausgeübt, so dass eine andrückende Verbindung des zweiten Formulierungselements 116 mit dem Formelement 10 erfolgt. Hierdurch wird die zweite Formulierung 50 in die leeren Bereiche der Formöffnungen 12' gedrückt.

Im dargestellten Bereich D sind somit die Formöffnungen 12" mit der zweiten Formulierung 50' befüllt. Somit sind die gesamten Formöffnungen 12 mit Formulierungen befüllt, die, insbesondere nach einem Aushärten, Mikrostrukturen entsprechen.

Fig. 4 zeigt ein Subsystem 103 eines Systems 100 zur Herstellung von Mikrostrukturen.

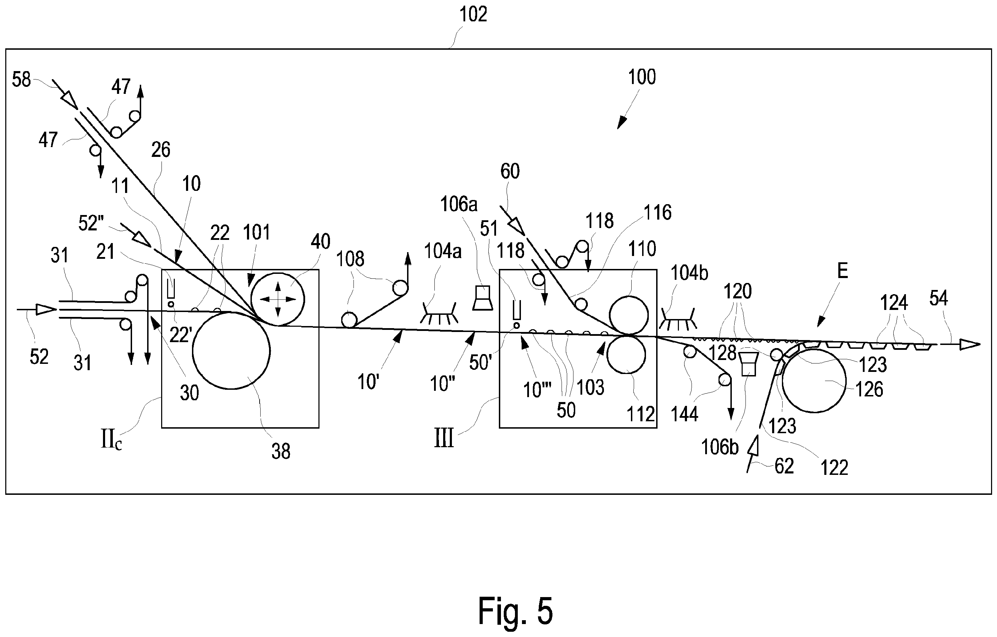

Fig. 5 zeigt eine Ausführungsform eines erfindungsgemäßen Systems 100 zur Herstellung von Mikrostrukturen.

Das System 100 ist in einem Gehäuse 102 angeordnet. Das Gehäuse ist vorzugsweise steril gegenüber der Umgebung. Zugeführte Elemente werden vorzugsweise vor der Zuführung sterilisiert und/oder durch Mouseholes in das Gehäuse 102 geführt. Bei dem Gehäuse 102 handelt es sich insbesondere um einen Isolator. Die Folien werden vorzugsweise mittels Verpackungsschlauch in das System eingeführt.

In Richtung des Pfeils 52' erfolgt die Zuführung eines ersten Formulierungselements 30, insbesondere mittels eines Verpackungsschlauchs 31. In Richtung des Pfeils 58 erfolgt eine Zuführung eines, vorzugsweise als Folie ausgebildeten, Hilfselements 26. Die Zuführung des Hilfselements 26 erfolgt insbesondere in einem Verpackungsschlauch 47.

Der im Kasten IIc dargestellte Bereich ist insbesondere entsprechend der Ausführung aus Fig. 2c oder Fig. 3 ausgeführt. Die Bereitstellung der ersten Formulierung 22 erfolgt hierbei über einen ersten Formulierungsdispenser 21, der die Formulierung 22 als Tröpfchen 22' auf das Formulierungselement 30, insbesondere in Vertiefungen 32 des Formulierungselements 30, aufträgt.

Bevorzugt erfolgt nach dem Bereich IIc eine Abführung des Hilfselements 26 über Rollen 108.

Anschließend erfolgt bevorzugt ein Trocknen der ersten Formulierung 22 im Formelement 10' über Trockenvorrichtung 104a.

Hieran schließt sich bevorzugt ein Prüfen des Formelements 10", insbesondere der vorzugsweise erstarrten Formulierung 22, an. Das Prüfen erfolgt vorzugsweise mittels Prüfvorrichtung 106a. Die Prüfvorrichtung ist insbesondere optisch ausgeführt. Besonders bevorzugt weist die Prüfvorrichtung mindestens eine Kamera auf.

Der sich anschließende Bereich III ist vorzugsweise entsprechend wie in Fig. 4 dargestellt ausgeführt. Die Bereitstellung der zweiten Formulierung 50 erfolgt insbesondere mittels eines zweiten Formulierungsdispensers 51, der die Formulierung 50 als Tröpfchen 50' auf das Formelement 10‴. aufträgt. Das zweite Formulierungselement 60 wird insbesondere im Rahmen eines Verpackungsschlauchs 118 durchgeführt. Das zweite Formulierungselement 60 weist insbesondere eine permeable und/oder feuchtigkeitsabsorbierende Folie auf, besteht insbesondere daraus.

Nach dem Bereich III erfolgt bevorzugt ein Abführen des Formelements 10 über Rollen 114. Besonders bevorzugt ist es hierbei, dass ebenfalls das erste Formulierungselement 30 mitabgeführt wird. Möglich ist es jedoch auch, dass das erste Formulierungselement 30 bereits vorher, irgendwann nach dem Bereich IIc, abgeführt wird. Beim Abführen des Formelements 10 erfolgt ein Entformen der vorzugsweise zu Mikrostrukturen 120 verbundenen Formulierungen 22, 50. Bevorzugt ist es hierbei, dass die Mikrostrukturen 120, beispielsweise adhäsiv, mit dem zweiten Formulierungselement verbunden sind und somit die Entformung und Weiterführung der Mikrostrukturen 120 erfolgt.

Vor und/oder während dem Entformen erfolgt bevorzugt eine Trocknung der vorzugsweise verbundenen Formulierungen 22, 50, die den herzustellenden Mikrostrukturen 120 entsprechen. Das Trocknen kann mittels Trockenvorrichtung 104b erfolgen. Zusätzlich oder alternativ kann jedoch auch eine Lufttrocknung durch die vorzugsweise permeable und/oder feuchtigkeitsabsorbierende Folie 60 erfolgen.

Bevorzugt erfolgt nach dem Entformen ein Prüfen der Mikrostrukturen 120. Es ist bevorzugt, dass das Prüfen mittels Prüfvorrichtung 106b erfolgt. Die Prüfvorrichtung weist insbesondere mindestens eine Kamera auf.

Anschließend erfolgt bevorzugt ein Verpacken der Mikrostrukturen 120. Hierzu wird bevorzugt eine Blisterfolie 122 in Richtung des Pfeils 62 zugeführt. Die Zuführung erfolgt insbesondere entlang Rollen 126, 128. Die Blisterfolie 122 weist mehrere nach oben geöffnete Blister 123 auf.

Im Bereich E erfolgt ein Verbinden der Blisterfolie 128 mit den Mikrostrukturen 120. Hierbei werden die Mikrostrukturen 120 in den Blistern 123 aufgenommen, so dass blisterverpackte Mikrostrukturen 124 vorliegen. Diese werden in Richtung des Pfeils 54 abgeführt.

## Patentansprüche

1. Verfahren zur Herstellung von Mikrostrukturen (120), insbesondere Mikroarrays, mit den Schritten:
Bereitstellen eines, mindestens eine Formöffnung (12) für die herzustellende Mikrostruktur (120) aufweisenden, flächigen Formelements (10), wobei die mindestens eine Formöffnung (12) eine erste Öffnung (14) und eine, insbesondere der ersten Öffnung (14) gegenüberliegende, zweite Öffnung (18) aufweist;
Bereitstellen einer ersten, vorzugsweise wirkstoffhaltigen, Formulierung (22) an der zweiten Öffnung (18);
Erzeugen eines Unterdrucks in der Formöffnung (12); und
Aufnehmen der ersten Formulierung (22) durch die zweite Öffnung (18) in die Formöffnung (12) aufgrund des Unterdrucks in der Formöffnung (12),
**gekennzeichnet durch**
einen weiteren Schritt vor dem Schritt des Erzeugens des Unterdrucks in der Formöffnung (12): Verringern des Volumens der Formöffnung (12),
wobei das Verringern des Volumens der Formöffnung (12) durch ein Komprimieren, insbesondere ein Zusammenpressen und/oder ein Biegen, des Formelements (10) erfolgt.

2. Verfahren nach Anspruch **1, gekennzeichnet durch** den Schritt: Erweitern eines Volumens der Formöffnung (12) zum Erzeugen des Unterdrucks in der Formöffnung (12).

3. Verfahren nach Anspruch 1 oder 2, **dadurch gekennzeichnet, dass** das Komprimieren des Formelements (10) mittels mindestens einer Walze (38; 40), vorzugsweise zwei beidseitig des Formelements (10) angeordneten Walzen (38, 40), erfolgt.

4. Verfahren nach einem der Ansprüche 1 bis 3, **gekennzeichnet durch** einen weiteren Schritt bevorzugt vor dem Schritt des Erzeugens des Unterdrucks in der Formöffnung (12), besonders bevorzugt vor dem Erweitern des Volumens:
Anordnen eines, insbesondere eine Hilfsfolie aufweisenden, Hilfselements (26), an einer, die erste Öffnung aufweisenden, Seite des Formelements (10).

5. Verfahren nach einem der Ansprüche 1 bis 4, **gekennzeichnet durch** einen weiteren Schritt vor dem Schritt des Erzeugens des Unterdrucks in der Formöffnung (12), insbesondere vor dem Komprimieren des Formelements (10): Verschließen der ersten Öffnung der Formöffnung (12), vorzugsweise mit dem Hilfselement (26).

6. Verfahren nach Anspruch 4, **gekennzeichnet durch** einen weiteren Schritt nach dem Aufnehmen der ersten Formulierung (22) in der Formöffnung (12):
Entfernen, insbesondere Abziehen, des Hilfselements (26) vorzugsweise vom Formelement (10).

7. Verfahren nach einem der Ansprüche 1 bis 6, **dadurch gekennzeichnet, dass** die mindestens eine Formöffnung (12) zylinderförmig oder kegelförmig, bevorzugt mit rundem, dreieckigem oder viereckigem, besonders bevorzugt quadratischem, Querschnitt ist; und/oder
dass das Formelement (10) eine Folie aufweist, insbesondere daraus besteht.

8. Verfahren nach einem der Ansprüche 1 bis 7, **dadurch gekennzeichnet, dass** das Formelement (10) komprimierbar, insbesondere elastisch komprimierbar, ist.

9. Verfahren nach einem der Ansprüche 1 bis 8, **gekennzeichnet durch** die weiteren Schritte nach dem Aufnehmen der ersten Formulierung (22):
Bereitstellen einer zweiten, vorzugsweise wirkstofffreien, Formulierung (50) an der ersten Öffnung (14); und
Aufnehmen der zweiten Formulierung (50) durch die erste Öffnung (14) in die Formöffnung (12), wobei sich die zweite Formulierung (50) vorzugsweise mit der ersten Formulierung (22) verbindet.

10. Verfahren nach einem der Ansprüche 1 bis 9, **dadurch gekennzeichnet, dass** das Bereitstellen der ersten Formulierung (22) mittels eines, vorzugsweise als Folie ausgebildeten, ersten Formulierungselements (30) erfolgt, wobei das ersten Formulierungselement (30) die erste Formulierung (22) aufweist; und/oder
dass das Bereitstellen der zweiten Formulierung (50) mittels eines, vorzugsweise als Folie ausgebildeten, zweiten Formulierungselements (116) erfolgt, wobei das zweiten Formulierungselement (116) die zweite Formulierung (50) aufweist.

11. Verfahren nach einem der Ansprüche 1 bis 10, **gekennzeichnet durch** einen weiteren Schritt: Entformen der zumindest teilweise zur Mikrostruktur (120) erstarrten ersten Formulierung (22) sowie vorzugsweise zweiten Formulierung (50) an der ersten Öffnung der Formöffnung (12), wobei es bevorzugt ist, dass
das Entformen mittels eines, vorzugsweise eine Deckfolie aufweisenden, Deckelements erfolgt, das mit der Formulierung, insbesondere stoffschlüssig Verbunden wird; und/oder dass
das Entformen durch ein Entfernen, insbesondere Abziehen, des Formelements (10) erfolgt.

12. Verfahren nach einem der Ansprüche 1 bis 11, **gekennzeichnet durch** einen weiteren Schritt: Verpacken der entformten Mikrostruktur (120), insbesondere mittels eines, vorzugsweise eine Blisterfolie aufweisenden, Blisterelements (122).

13. Verfahren nach einem der Ansprüche 1 bis 12, **dadurch gekennzeichnet, dass** das Verfahren mit einem System (100) nach Anspruch 18 oder 19 durchgeführt wird.

14. System (100) zur Herstellung von Mikrostrukturen (120), insbesondere zur Durchführung eines Verfahrens nach einem der Ansprüche einem der Ansprüche 1 bis 13, mit
einem, mindestens eine Formöffnung (12) für die herzustellende Mikrostruktur (120) aufweisenden, komprimierbaren Formelement, und
einer, mindestens eine Walze (38; 40) aufweisenden, Komprimiervorrichtung (37),
wobei die Komprimiervorrichtung zum Komprimieren des Formelements (10) ausgeführt ist.

15. System nach Anspruch 14, **dadurch gekennzeichnet, dass** die Komprimiervorrichtung (37) zwei sich gegenüberliegende Walzen (38, 40) aufweist, wobei die Walzen (38, 40) derart angeordnet sind, dass das Formelement (10) zwischen den Walzen (38, 40) komprimierbar ist.

## Claims

1. A method for producing microstructures (120), in particular microarrays, the method comprising the following steps: providing a planar mold element (10), which comprises at least one mold opening (12) for the microstructure (120) to be produced, the at least one mold opening (12) having a first opening (14) and a second opening (18) which, in particular, is located opposite the first opening (14); providing a first formulation (22) at the second opening (18), said formulation preferably containing an active ingredient; generating a negative pressure in the mold opening (12); and taking up the first formulation (22) through the second opening (18) into the mold opening (12) on account of the negative pressure in the mold opening (12), **characterized by** a further step prior to the step of generating the negative pressure in the mold opening (12): reducing the volume of the mold opening (12), wherein reducing the volume of the mold opening (12) is effected by a compression, in particular a pressing and/or bending, of the mold element (10).

2. The method of claim 1, **characterized by** the step of: expanding a volume of the mold opening (12) to generate the negative pressure in the mold opening (12).

3. The method according to claim 1 or 2, **characterized in that** the compression of the mold element (10) is performed using at least one roll (38; 40), preferably two rolls (38, 40) arranged on either side of the mold element (10).

4. The method according to any one of claims 1 to 3, **characterized by** a further step, preferably prior to the step of generating the negative pressure in the mold opening (12), particularly preferred prior to the expansion of the volume:
arranging an auxiliary element (26), in particular comprising an auxiliary film, on a side of the mold element (10) having the first opening.

5. The method according to any one of claims 1 to 4, **characterized by** a further step, preferably prior to the step of generating the negative pressure in the mold opening (12), particularly preferred prior to the compression of the mold element (10): closing the first opening of the mold opening (12), preferably by means of the auxiliary element (26).

6. The method according to claim 4, **characterized by** a further step after the taking-up of the first formulation (22) into the first mold opening (12):
removing, in particular pulling off the auxiliary element (26) preferably from the mold element (10).

7. The method according to any one of claims 1 to 6, **characterized in that** the at least one mold opening (12) is cylindrical or conical, preferably with a round, triangular or quadrangular, particularly preferred square cross section, and/or
the mold element (10) comprises, in particular consists of a film.

8. The method according to any of claims 1 to 7, **characterized in that** the mold element (10) is compressible, in particular elastically compressible.

9. The method according to any of claims 1 to 8, **characterized by** the further steps after the taking-up of the first formulation (22):
providing a second formulation (50) at the first opening (14), said second formulation preferably being free of an active ingredient; and
taking up the second formulation (50) into the mold opening (12) through the first opening (14), the second formulation (50) preferably bonding with the first formulation (22).

10. The method according to any one of claims 1 to 9, **characterized in that** providing the first formulation (22) is performed using a first formulation element (30) preferably designed as a film, the first formulation element (30) comprising the first formulation (22); and/or
that providing the second formulation (50) is performed using a second formulation element (116) preferably designed as a film, the second formulation element (116) comprising the second formulation (50).

11. The method according to any one of claims 1 to 10, **characterized by** a further step: demolding the first formulation (22), which has at least partially solidified to form a microstructure (120), and preferably the second formulation (50) at the first opening of the mold opening (12), it being preferred that
the demolding is performed using a cover element preferably comprising a cover film, which cover element is in particular substance-bonded with the formulation; and/or
the demolding is performed by removing, in particular pulling off the mold element (10).

12. The method according to any one of claims 1 to 11, **characterized by** a further step: packaging the demolded microstructure (120) using, in particular, a blister element (122) preferably comprising a blister film.

13. The method according to one of claims 1 to 12, **characterized in that** the method is performed using a system (100) according to claim 14 or 15.

14. A system (100) for producing microstructures (120), in particular for executing a method according to one of claims 1 to 13, comprising
a compressible mold element comprising at least one mold opening (12) for the microstructure (120) to be produced, and
a compression device (37) preferably comprising at least one roll (38; 40),
the compression device being configured to compress the mold element (10).

15. The system according to claim 14, **characterized in that** the compression device (37) comprises two opposite rolls (38, 40), the rolls (38, 40) being arranged such that the mold element (10) can be compressed between the rolls (38, 40).

## Revendications

1. Procédé de fabrication de microstructures (120), en particulier de microréseaux, comportant les étapes consistant à :
fournir un élément de moulage (10) plat présentant au moins une ouverture de moulage (12) pour la microstructure (120) à fabriquer, dans lequel l'au moins une ouverture de moulage (12) présente une première ouverture (14) et une deuxième ouverture (18), en particulier opposée à la première ouverture (14) ;
fournir une première formulation (22), de préférence contenant une substance active, au niveau de la deuxième ouverture (18) ;
générer une dépression dans l'ouverture de moulage (12) ; et
recevoir la première formulation (22) à travers la deuxième ouverture (18) dans l'ouverture de moulage (12) en raison de la dépression dans l'ouverture de moulage (12), **caractérisé par**
une étape supplémentaire précédant l'étape de génération de la dépression dans l'ouverture de moulage (12) : réduction du volume de l'ouverture de moulage (12),
dans lequel la réduction du volume de l'ouverture de moulage (12) est effectuée par une compression, en particulier un resserrage et/ou un pliage, de l'élément de moulage (10).

2. Procédé selon la revendication 1, **caractérisé par** l'étape consistant à : augmenter un volume de l'ouverture de moulage (12) pour la génération de la dépression dans l'ouverture de moulage (12).

3. Procédé selon la revendication 1 ou 2, **caractérisé en ce que** la compression de l'élément de moulage (10) est effectuée au moyen d'au moins un rouleau (38 ; 40), de préférence de deux rouleaux (38, 40) disposés de part et d'autre de l'élément de moulage (10).

4. Procédé selon l'une des revendications 1 à 3, **caractérisé par** une étape supplémentaire, de préférence précédant l'étape de génération de la dépression dans l'ouverture de moulage (12), de manière particulièrement préférée précédant l'augmentation du volume :
disposition d'un élément auxiliaire (26), présentant en particulier une feuille auxiliaire, sur un côté de l'élément de moulage (10) présentant la première ouverture.

5. Procédé selon l'une des revendications 1 à 4, **caractérisé par** une étape supplémentaire précédant l'étape de génération de la dépression dans l'ouverture de moulage (12), en particulier précédant la compression de l'élément de moulage (10) : fermeture de la première ouverture de l'ouverture de moulage (12), de préférence avec l'élément auxiliaire (26).

6. Procédé selon la revendication 4, **caractérisé par** une étape supplémentaire succédant à la réception de la première formulation (22) dans l'ouverture de moulage (12) :
élimination, en particulier retrait, de l'élément auxiliaire (26), de préférence depuis l'élément de moulage (10).

7. Procédé selon l'une des revendications 1 à 6, **caractérisé en ce que** l'au moins une ouverture de moulage (12) est de forme cylindrique ou conique, de préférence avec une section transversale ronde, triangulaire ou quadrangulaire, de manière particulièrement préférée carrée ; et/ou
**en ce que** l'élément de moulage (10) présente une feuille, en particulier en est constitué.

8. Procédé selon l'une des revendications 1 à 7, **caractérisé en ce que** l'élément de moulage (10) est compressible, en particulier est compressible de manière élastique.

9. Procédé selon l'une des revendications 1 à 8, **caractérisé par** les étapes supplémentaires succédant à la réception de la première formulation (22) : fourniture d'une deuxième formulation (50), de préférence sans principe actif, au niveau de la première ouverture (14) ; et
réception de la deuxième formulation (50) à travers la première ouverture (14) dans l'ouverture de moulage (12), dans lequel la deuxième formulation (50) se combine de préférence avec la première formulation (22).

10. Procédé selon l'une des revendications 1 à 9, **caractérisé en ce que** la fourniture de la première formulation (22) est effectuée au moyen d'un premier élément à formulation (30) de préférence réalisé sous forme de feuille, dans lequel le premier élément à formulation (30) présente la première formulation (22) ; et/ou
**en ce que** la fourniture de la deuxième formulation (50) est effectuée au moyen d'un second élément à formulation (116) de préférence réalisé sous forme de feuille, dans lequel le second élément à formulation (116) présente la deuxième formulation (50).

11. Procédé selon l'une des revendications 1 à 10, **caractérisé par** une étape supplémentaire : démoulage de la première formulation (22) au moins partiellement solidifiée en microstructure (120) ainsi que, de préférence, de la deuxième formulation (50) au niveau de la première ouverture de l'ouverture de moulage (12), dans lequel de préférence
le démoulage est effectué au moyen d'un élément de recouvrement présentant de préférence une feuille de recouvrement, lequel élément de recouvrement est relié à la formulation, en particulier par liaison de matière ; et/ou en ce que
le démoulage est effectué par une élimination, en particulier un retrait, de l'élément de moulage (10).

12. Procédé selon l'une des revendications 1 à 11, **caractérisé par** une étape supplémentaire : emballage de la microstructure (120) démoulée, en particulier au moyen d'un élément à emballage-coque (122) présentant de préférence une feuille formant emballage-coque.

13. Procédé selon l'une des revendications 1 à 12, **caractérisé en ce que** le procédé est mis en œuvre avec un système (100) selon la revendication 18 ou 19.

14. Système (100) de fabrication de microstructures (120), en particulier pour la mise en œuvre d'un procédé selon l'une des revendications 1 à 13, comportant
un élément de moulage compressible présentant au moins une ouverture de moulage (12) pour la microstructure (120) à fabriquer, et un dispositif de compression (37) présentant au moins un rouleau (38 ; 40),
dans lequel le dispositif de compression est conçu pour comprimer l'élément de moulage (10).

15. Système selon la revendication 14, **caractérisé en ce que** le dispositif de compression (37) présente deux rouleaux (38, 40) opposés, dans lequel les rouleaux (38, 40) sont disposés de telle sorte que l'élément de moulage (10) peut être comprimé entre les rouleaux (38, 40).
